# EUROPÄISCHE PATENTANMELDUNG

(11) **EP 1 138 304 A2**
(43) Veröffentlichungstag der Anmeldung: **04.10.2001**
(21) Anmeldenummer: 01104980.6
(22) Anmeldetag: 01.03.2001
(51) Int. Cl.: A61G 12/00

(54) **Unterschrank für medizinische Geräte**

(30) Priorität: 30.03.2000 DE 20005770 U
(71) Anmelder: Barkey, Volker, D-33619 Bielefeld (DE)
(72) Erfinder: Barkey, Volker, D-33619 Bielefeld (DE)
(74) Vertreter: TER MEER STEINMEISTER & PARTNER GbR

(57) **Zusammenfassung**

Bei einem Unterschrank für medizinische Geräte, insbesondere für Auftau- und Temperiergeräte für gekühlte oder tiefgekühlte medizinische Produkte, Temperiergeräte für Babynahrung und dergleichen weist der Unterschrank eine Anzahl von übereinanderliegenden Fachböden oder Schubkästen (10,12,14) auf, deren Boden (28,30,32) aus Lochplatten, Gittergewebe oder dergleichen luftdurchlässigen Materialien besteht. In dem Unterschrank ist eine geregelte Heizeinrichtung (46,48) vorgesehen.

## Beschreibung

Die Erfindung betrifft einen Unterschrank für medizinische Geräte, insbesondere für Auftau- und Temperiergeräte für gekühlte oder tiefgekühlte medizinische Produkte, Temperiergeräte für Babynahrung und dergleichen.

Im Betrieb eines Krankenhauses werden häufig Operations-Patienten, Unfallopfer und dergleichen mit Blut- oder Plasmakonserven, Infusions-Nährlösungen und anderen medizinischen Flüssigkeiten versorgt, die gekühlt oder sogar tiefgekühlt gelagert werden und daher zunächst aufgetaut und/oder erwärmt werden müssen. In Entbindungsstationen ist die Verabreichung von angewärmter Babynahrung erforderlich. Es gibt daher zahlreiche Fälle, in denen medizinische Produkte im weitesten Sinne aufgetaut, erwärmt oder warmgehalten werden müssen. Es sind verschiedene Auftau- und Temperiergeräte bekannt, die dazu dienen, die genannten Produkte auf die erforderliche Verabreichungstemperatur, beispielsweise Körpertemperatur zu bringen. In diesen Geräten erfordert die Temperierung jedoch eine gewisse Zeit, so daß es zu einem Rückstau kommen kann, wenn beispielsweise mehrere Unfallopfer versorgt werden sollen.

Der Erfindung liegt daher die Aufgabe zugrunde, ein Hilfsmittel zu schaffen, das es gestattet, die Kapazität von Temperiereinrichtungen aller Art zu erhöhen.

Diese Aufgabe wird erfindungsgemäß durch einen Unterschrank der eingangs genannten Art gelöst, der dadurch gekennzeichnet ist, daß der Unterschrank eine Anzahl von übereinanderliegenden Fachböden oder Schubkästen aufweist, deren Boden aus Lochplatten, Gittergewebe oder dergleichen luftdurchlässigen Materialien besteht, und daß in dem Unterschrank oder unmittelbar an dem Unterschrank eine geregelte Heizeinrichtung vorgesehen ist.

Der erfindungsgemäße Unterschrank bietet daher nicht nur eine passende Stellfläche für Temperiergeräte der dargestellten Art, sondern einen Aufnahmeraum, in dem die in überschaubarer Zeit benötigten, temperierungsbedürftigen Produkte vorgewärmt werden können.

Zu diesem Zweck ist der Unterschrank durch alle Fachböden oder Schubkasten-Böden hindurch durchlässig für einen Warmluftstrom, so daß geheizte und in ihrer Temperatur geregelte Warmluft in dem Schrank zirkulieren kann.

Vorzugsweise ist eine Heizeinrichtung in Verbindung mit einem Gebläse vorgesehen, das die Luft in dem Unterschrank in Bewegung hält und für eine gleichmäßige Durchmischung der Temperaturbereiche sorgt.

Zweckmäßigerweise befindet sich auf der Innenseite vor der Rückwand eine Zwischenwand, hinter der die Heizeinrichtung mit dem Gebläse angeordnet ist.

Der Unterschrank kann mit Rollen versehen sein, so daß er zusammen mit der Temperiervorrichtung an den Einsatzort verschoben werden kann. Er bietet auf diese Weise noch eine zusätzliche Funktion neben dem Erwärmen der Produkte und der Bildung einer Standfläche für das Temperiergerät.

Vorzugsweise ist eine elektronische Temperaturregelung vorgesehen, die beispielsweise die Lufttemperatur am Ausgang der Heizeinrichtung bzw. des Gebläses misst und eine entsprechende Regelung vornimmt. Die erwärmte Luft sollte unterhalb des unteren Bodens oder des unteren Schubkastens zugeführt werden und durch die einzelnen Schubkästen aufsteigen können.

Die inneren Ecken und Kanten des Unterschranks können zur Verbesserung der Luftumwälzung strömungsgünstig ausgerundet sein. Zugleich können die oberen, unteren und seitlichen Außenwände des Unterschranks aus einem wärmedämmenden oder wärmeisolierenden Material bestehen, damit die innere Temperatur mit geringer zusätzlicher Heizung gehalten werden kann. Wenn der Unterschrank oder eine seiner Schubkästen geöffnet wird, sollte das Gebläse automatisch abgeschaltet werden, damit der Ausstoß warmer Luft und dadurch entstehende Wärmeverluste vermieden werden.

An dem Unterschrank kann ein Infusionsständer zum Aufhängen eines Infusionsbeutels oder einer Infusionsflasche vorgesehen sein, so daß der erfindungsgemäße Unterschrank zusammen mit einer geeigneten Temperiereinrichtung und dem Infusionsständer die Funktion eines verfahrbaren Infusionszentrums erhält.

In diesem Sinne können auch Griffe an der vorderen oder rückwärtigen Seite des Unterschranks zum Lenken und Schieben des Unterschranks angebracht sein.

Vorzugsweise befinden sich an dem Unterschrank Steckdosen für weitere Verbraucher, die zusammen mit der Heizeinrichtung über eine gemeinsame Zuleitung gespeist werden.

Weitere Merkmale und Vorteile der Erfindung ergeben sich aus den Unteransprüchen.

Im folgenden werden bevorzugte Ausführungsbeispiele der Erfindung anhand der beigefügten Zeichnung näher erläutert.
- Fig. 1: ist eine perspektivische Darstellung eines erfindungsgemäßen Unterschranks;
- Fig. 2: ist eine schematische Seitenansicht, der teilweise als senkrechter Schnitt dargestellt ist;
- Fig. 3: ist eine schematische Darstellung einer anderen Ausführungsform der Erfindung.

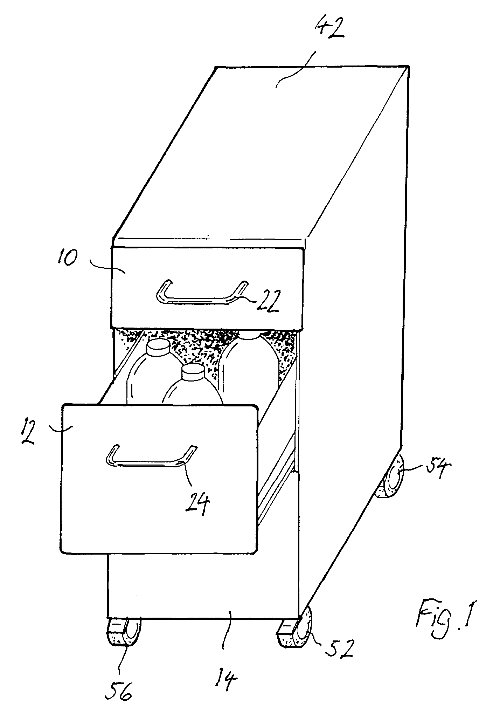

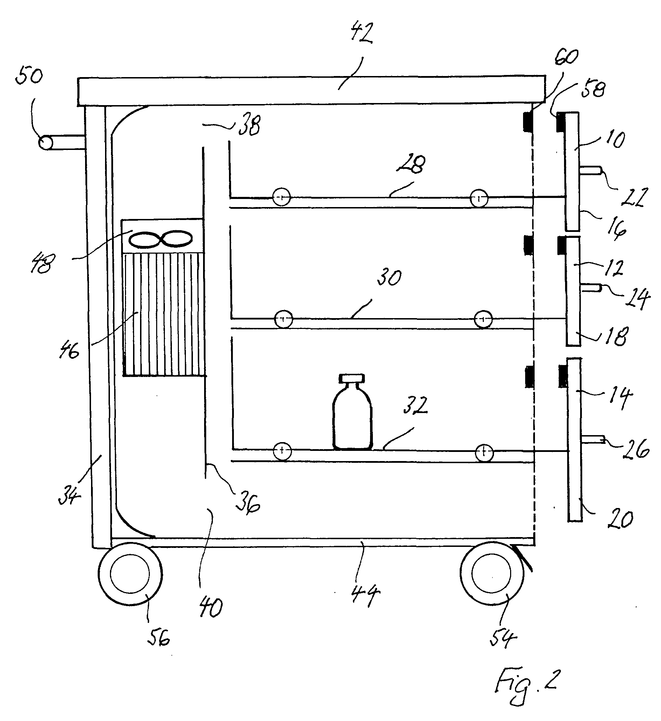

Fig. 1 und 2 zeigen einen erfindungsgemäßen Unterschrank, der mit drei übereinanderliegenden Schubkästen 10,12,14 ausgestattet ist. Diese Schubkästen weisen übliche, nicht bezeichnete Rollenführungen auf. An den Frontplatten 16,18,20 der Schubkästen befinden sich Handgriffe 22,24,26. Die Böden 28,30,32 der Schubkästen bestehen aus einem Lochblech, einem Gittergewebe oder dergleichen, in jedem Falle einem weitgehend luftdurchlässigen Material, das eine intensive Luftumwälzung in dem Unterschrank ermöglicht.

Zwischen der der Auszugsseite der Schubkästen gegenüberliegenden Rückwand 34 und einer parallel im Abstand zu dieser Rückwand im Inneren des Unterschranks liegenden Zwischenwand 36, der obere und untere Luftdurchlässe 38,40 gegenüber einer oberen Abdeckplatte 42 und einem unteren Boden 44 freiläßt, befindet sich eine Heizeinrichtung 46 mit einem Gebläse 48. Mit Hilfe dieser Heizeinrichtung kann beispielsweise warme Luft durch den unteren Luftdurchlaß 40 in den von den Schubkästen 10,12,14 eingenommenen Raum geblasen werden, die dann durch die Schubkästen und die in diesen befindlichen Produkte hindurch aufsteigt. Durch diese Luftzirkulation können die in dem Unterschrank aufbewahrten Produkte gleichmäßig erwärmt werden.

An der Außenseite der Rückwand 34 befindet sich ein Handgriff 50, und unterhalb des Bodens 44 sind an dem Unterschrank Rollen 52,54,56 gelagert, so daß der Unterschrank nicht nur zum Abstützen eines auf der Abdeckplatte 42 abzustellenden Temperiergeräts, sondern als Transportmittel für dieses verwendet werden kann. Ein Handgriff kann auch auf der gegenüberliegenden Seite, etwa an der Abdeckplatte 42 oberhalb der Schubkästen vorgesehen sein.

An der Innenseite der Frontplatten 16,18,20 der Schubkästen befinden sich Eisenplatten 58, die mit Magneten 60 auf der Seite des Unterschranks zusammenwirken und die Schubkästen geschlossen halten, so daß die Temperatur im Inneren des Unterschranks so weit wie möglich erhalten bleiben. Die Magnete können auch an der Rückseite der Schubkästen einerseits und der Zwischenwand 36 andererseits angebracht sein.

Anstelle von Schubkästen können auch offene Fächer mit luftdurchlässigen Fachböden vorgesehen sein.

Vorzugsweise ist das Gebläse 48 der Heizeinrichtung so angeordnet, daß die warme Luft zum Boden des Unterschranks bewegt wird und von hier aus durch die einzelnen Fachböden aufsteigt.

Die Zwischenwand 36 erstreckt sich nicht zwangsläufig über die gesamte Breite zwischen den Seitenwänden, sondern kann auch als senkrechtes Gehäuse ausgebildet sein, das an der Rückwand angebracht ist.

Innerhalb des Unterschranks ist ein nicht dargestelltes Thermometer vorgesehen, das eine thermostatische Regelung der Heizeinrichtung ermöglicht.

Die Heizung kann mit dem Gebläse als zusammenhängende Einheit an der Rückwand montiert sein.

Bei der zuvor beschriebenen Ausführungsform ist davon ausgegangen worden, daß die Heizeinrichtung zwischen der Rückwand des Unterschranks und einer parallel und in Abstand zu der Rückwand liegenden Zwischenwand angeordnet sein soll. Dies ist nicht zwangsläufig der Fall. Grundsätzlich ist es auch möglich, die Heizeinrichtung oben, unten oder an einer der Seitenwände anzuordnen.

Eine Anordnung an einer Seitenwand bietet sich insbesondere dann an, wenn in Richtung der Tiefe des Unterschranks nicht ausreichend Platz zur Verfügung steht. Dies kann bei Unterschränken der Fall sein, die als sogenanntes Einbaugerät verwendet werden sollen, das beispielsweise unterhalb einer Arbeitsplatte angeordnet werden soll.

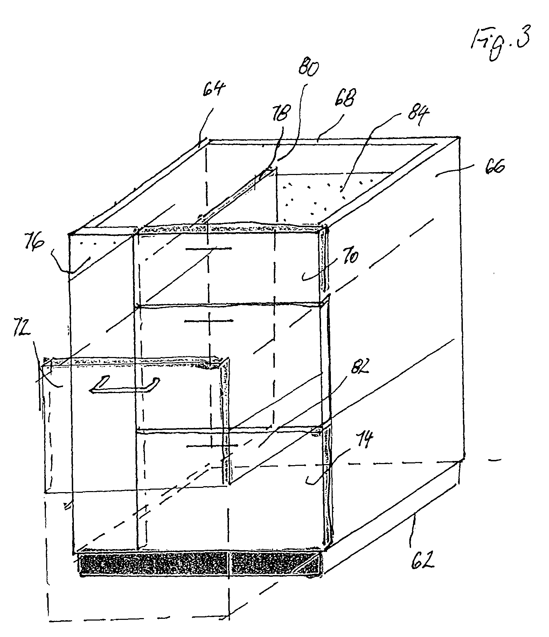

Ein derartiger Unterschrank ist in Fig. 3 angedeutet worden. Ein Unterschrank gemäß Fig. 3 umfaßt einen Boden 62, eine linke und eine rechte Seitenwand 64,66 und eine Rückwand 68. Auch in diesem Falle sind drei zum Betrachter hin ausziehbare Schubkästen 70,72,74 vorgesehen. Links von den Schubkästen 70,72,74 in Fig. 3 befindet sich eine schmale Frontplatte 76. In dem Raum hinter der Frontplatte 76 befindet sich eine nicht im einzelnen dargestellte Heizeinrichtung, die zu den Schubkästen 70,72,74 hin durch eine Zwischenwand 78 abgetrennt ist.

Wie bei der Ausführungsform gemäß Fig. 1 und 2, läßt die Zwischenwand 78 nach oben und unten zum Boden 62 hin Luftdurchlässe 80,82 frei. Es ist daher möglich, mit Hilfe der nicht dargestellten Heizeinrichtung und eines dieser zugeordneten, ebenfalls nicht dargestellten Gebläses eine Luftzirkulation in dem Schrank zu erzeugen, die beispielsweise von unten durch die Siebböden der Schubkästen 70,72,74 aufsteigt und oben über die Zwischenwand 78 durch den Luftdurchlaß 80 in den Bereich der Heizeinrichtung zurückkehrt.

Die Frontplatte 76 kann mit Hilfe von Scharnieren schwenkbar an der Seitenwand 64 angebracht, auf Führungen herausziehbar oder auch vollständig abnehmbar sein, so daß der Raum hinter der Frontplatte, in dem sich die Heizeinrichtung und das Gebläse befinden, von vorne zugänglich wird. Dies kann beispielsweise für Wartungs- und Reparaturarbeiten an der Heizeinrichtung sinnvoll sein.

Der gesamte Unterschrank kann aus Holzwerkstoff-Materialien, etwa sogenannten MDF- oder HDF-Platten bestehen. Er kann aber auch ganz oder teilweise aus Blech angefertigt sein. Insbesondere die Rückwand kann teilweise als Blechplatte 84 ausgebildet sein.

An geeigneter Stelle, beispielsweise auf der genannten Blechplatte können Endschalter vorgesehen sein, die die Heizeinrichtung abschalten, wenn ein Schubkasten ausgezogen wird.

Durch zusätzliche Maßnahmen kann sichergestellt sein, daß der Inhalt aller Schubkästen im wesentlichen gleich erwärmt wird. So kann beispielsweise das Gebläse in der Drehrichtung umschaltbar sein, so daß die erwärmte Luft zeitweilig von unten in die Schubkästen eintritt und zeitweilig von oben eingeleitet wird. Es ist auch möglich, Leitbleche zur Verbesserung der gleichmäßigen Luftverteilung vorzusehen oder auch in der Zwischenwand 78 Schlitze anzubringen.

## Patentansprüche

1. Unterschrank für medizinische Geräte, insbesondere für Auftau- und Temperiergeräte für gekühlte oder tiefgekühlte medizinische Produkte, Temperiergeräte für Babynahrung und dergleichen, **dadurch gekennzeichnet, daß** der Unterschrank eine Anzahl von übereinanderliegenden Fachböden oder Schubkästen (10,12,14;70,72,74) aufweist, deren Boden (28,30,32) aus Lochplatten, Gittergewebe oder dergleichen luftdurchlässigen Materialien besteht, und **daß** in dem Unterschrank eine geregelte Heizeinrichtung (46,48) vorgesehen ist.

2. Unterschrank nach Anspruch 1, **dadurch gekennzeichnet, daß** die Heizeinrichtung wenigstens ein Gebläse (48) umfaßt.

3. Unterschrank nach Anspruch 1 oder 2, **dadurch gekennzeichnet, daß** der Unterschrank Rollen (52,54,56) aufweist.

4. Unterschrank nach den Ansprüchen 1 bis 3, **dadurch gekennzeichnet, daß** die Heizeinrichtung (46,48) zwischen einer Außenwand (34) und einer im Inneren des Unterschranks liegenden, parallel zu dieser Außenwand verlaufenden Zwischenwand (36;78) angeordnet ist, die Luftdurchlässe (38,40;80,82)) zu dem übrigen Innenraum des Unterschranks freiläßt.

5. Unterschrank nach einem der Ansprüche 1 bis 4, **dadurch gekennzeichnet, daß** eine elektronische Temperaturregelung vorgesehen ist, die die Lufttemperatur am Austritt der Heizeinrichtung (46,48) erfaßt.

6. Unterschrank nach Anspruch 5, **dadurch gekennzeichnet, daß** die Heizeinrichtung (46,48) einen nach unten zum Boden (44;62) des Unterschranks gerichteten Warmluftstrom abgibt, derart, **daß** die Warmluft von der Heizeinrichtung aus von unten nach oben durch die Böden (28,30,32) strömt.

7. Unterschrank nach einem der vorhergehenden Ansprüche, **dadurch gekennzeichnet, daß** der gesamte Korpus des Unterschranks aus wärmedämmenden Material besteht.

8. Unterschrank nach einem der vorhergehenden Ansprüche, **dadurch gekennzeichnet, daß** die Rückwände der Schubkästen (10,12,14) aus luftdurchlässigem Material bestehen.

9. Unterschrank nach einem der vorhergehenden Ansprüche, **dadurch gekennzeichnet, daß** die Heizeinrichtung (46,48) beim Öffnen des Unterschranks automatisch abschaltbar ist.

10. Unterschrank nach einem der vorhergehenden Ansprüche, **dadurch gekennzeichnet, daß** Haltemagnete (58,60) zum Festhalten der Schubkästen (10,12,14) in der geschlossenen Stellung vorgesehen sind.

11. Unterschrank nach einem der vorhergehenden Ansprüche, **dadurch gekennzeichnet, daß** am Unterschrank ein Handgriff (50) vorgesehen ist.

12. Unterschrank nach einem der vorhergehenden Ansprüche, **dadurch gekennzeichnet, daß** für den Unterschrank an der Außenseite wenigstens eine Steckdose vorgesehen ist, die mit der gemeinsamen elektrischen Zuleitung von Heizeinrichtung (46,48) und Gebläse (48) verbunden ist.

13. Unterschrank nach einem der Ansprüche 4 bis 12, **dadurch gekennzeichnet, daß** die Heizeinrichtung (46,48) zwischen der Rückwand (34) und einer parallel und im Abstand zu der Rückwand liegenden Zwischenwand (36) angeordnet ist.

14. Unteranschrank nach einem der Ansprüche 4 bis 12, **dadurch gekennzeichnet, daß** die Heizeinrichtung (46,48) zwischen einer Seitenwand (64) und einer parallel und im Abstand zu dieser liegenden Zwischenwand (78) im Inneren des Unterschranks angeordnet ist und **daß** der von der Heizeinrichtung eingenommene Raum im Inneren des Unterschranks zur Frontseite hin durch eine Frontplatte (76) abgedeckt ist.

15. Unterschrank nach Anspruch 14, **dadurch gekennzeichnet, daß** die Frontplatte (76) aufklappbar oder abnehmbar ist.

16. Unterschrank nach Anspruch 15, **dadurch gekennzeichnet, daß** die Heizeinrichtung (46,48) nach vorne herausnehmbar ist.
